# EUROPEAN PATENT SPECIFICATION

(11) **EP 3 479 852 B1**
(45) Date of publication and mention of the grant of the patent: **31.05.2023**
(21) Application number: 18159544.8
(22) Date of filing: 01.03.2018
(51) Int. Cl.: A61L 27/50, A61L 27/56, A61L 27/58

(54) **A POROUS BIPHASIC SCAFFOLD FOR OSTEOCHONDRAL COMPOSITE TISSUE REGENERATION AND A MANUFACTURING METHOD THEREOF**
PORÖSES, ZWEIPHASIGES GERÜST ZUR OSTEOCHONDRALEN VERBUNDGEWEBEREGENERATION UND HERSTELLUNGSVERFAHREN DAFÜR
ÉCHAFAUDAGE BIPHASIQUE POREUX POUR LA RÉGÉNÉRATION DE TISSUS COMPOSITES OSTÉOCHONDRAUX ET SON PROCÉDÉ DE FABRICATION

(30) Priority: 03.11.2017 KR 20170146275
(43) Date of publication of application: 08.05.2019
(73) Proprietor: Dankook University Cheonan Campus Industry Academic Cooperation Foundation, Chungcheongnam-do 31116 (KR)
(72) Inventor: KIM, Hae-Won, Cheonan-si, Chungcheongnam-do 31170 (KR); KIM, Jung Ju, Cheonan-si, Chungcheongnam-do 31109 (KR); MAHAPATRA, Chinmaya, Cheonan-si, Chungcheongnam-do 31116 (KR); CAMPBELL KNOWLES, Jonathan, St Albans, Hertfordshire AL3 5AJ (GB)
(74) Representative: Regimbeau

(56) References cited:
- WO-A2-2012/046957
- US-A1- 2014 012 393

## Description

### [Technical Field]

The present invention relates to a porous biphasic scaffold for osteochondral composite tissue regeneration embodied in an integrated form having a structure in which a dense scaffold and a nano fibrous scaffold are laminated, and a manufacturing method thereof.

### [Background Art]

Regeneration of a complex tissue including osteochondral interface has presented significant challenges in orthopedic and maxillofacial operations. Despite reports of promising results, there have not been many studies related to manipulating interfaces. This is because the cells and the constituents of the tissues constituting the interfacial structure are very complicated. An osteochondral composite tissue is a permanent interfacial structure between bone and cartilage. The osteochondral composite tissue is defined as a thin layer of calcified cartilage between uncalcified hyaline cartilage and subchondral bone. Among its physical properties, progressive deformation can lower the concentration of stress at the interface and also prevent damage. In terms of such mechanical considerations, the interface has a unique biochemical composition containing a high concentration of calcium salts and vertically moving collagen fibrils.

As such, it is necessary to develop a suitable biomaterial that can be introduced to a defect at the osteochondral interface and functionally harmonize the complex tissue structures. In other words, a bio-functionally or multi-functionally structured biomaterial designed to mimic individual tissue structures and/or stimulate functions of each cell type is required. A scaffold for use in the osteochondral region is designed to regenerate bone and cartilage. Meanwhile, several research groups have suggested use of a biphasic scaffold for recovery of an osteochondral tissue. The patent application US 2014/012393 describes a complex support body for regerating bone-cartilage, which comprises a bone regeneration layer consisting of a biodegradable polymer and a biocompatible ceramic, and a cartilage regeneration layer, which is coupled to the bone regeneration layer. The patent application WO 2012/046957 describes a preparation method of a medical biopolymer having a nanofiber structure using camphene as a reverse phase and a method for preparing a 3D scaffold having a nano fiber structure by utilizing camphene as a reverse phase.

Meanwhile, a dissertation (Kazunori Shimomura, [Osteochondral Tissue Engineering with Biphasic Scaffold: Current Strategies and Techniques](TISSUE ENGINEERING: Part B, 2014)) discloses manufacture of a biphasic scaffold for the treatment of osteochondral lesion; the constitution of a biphasic scaffold in which separately prepared "cartilage layer scaffold" and "subchondral bone scaffold" were combined in layers. The effect of osteocyte and chondrocytes growth on each scaffold was insignificant, and no research developments have been made to alter the structure of the scaffold so as to make it useful for cell differentiation or maintenance of cellular phenotypes.

Under such circumstances, the present inventors endeavored to manufacture a structure facilitating effective and economical mass production for osteochondral composite tissue regeneration, and as a result, have developed a method for preparing a scaffold in which dense and nanofibrous surfaces are embodied in an integrated form using phase separation, thereby completing the present invention.

### [Disclosure]

### [Technical Problem]

An object of the present disclosure relates to providing a porous biphasic scaffold for osteochondral composite tissue regeneration, comprising a first layer scaffold having a dense structure for cartilage regeneration comprising a biodegradable polymer; and a second layer scaffold having a nanofibrous structure for bone regeneration comprising a biodegradable polymer and camphene.

Another object of the present disclosure is to provide a manufacturing method of the porous biphasic scaffold for osteochondral composite tissue regeneration.

### [Technical Solution]

A first aspect of the present invention provides a porous biphasic scaffold for osteochondral composite tissue regeneration, comprising a first layer scaffold having a dense structure for cartilage regeneration comprising a biodegradable polymer; and a second layer scaffold having a nanofibrous structure for bone regeneration comprising a biodegradable polymer, wherein the nanofibrous structure of the second layer scaffold is created by the use of camphene and wherein the surface area of the first layer scaffold is 2 m²/g to 4 m²/g and that of the second layer scaffold is 12 m²/g to 14 m²/g.

A second aspect of the present invention provides a method for preparing a porous biphasic scaffold for osteochondral composite tissue regeneration, comprising (i) preparing a biodegradable polymer slurry to which camphene is added and a biodegradable polymer slurry to which camphene is not added; (2) preparing a mold in which metal salt particles of a uniform size are present; (3) injecting one of the slurries prepared in the first step into the mold prepared in the second step followed by lyophilizing; and (4) injecting the other slurry into the mold followed by lyophilizing.

Hereinbelow, the present invention will be described in detail.

In order to discover a material simultaneously facilitating differentiation of osteocytes and chondrocytes in an osteochondral composite tissue, the present invention is based on designing an integrated form of a scaffold in which the osteocytes are well differentiated and a porous scaffold in which the chondrocytes are well differentiated, and uses a principle that cell-to-cell or cell-to-scaffold affinity determines proliferation of the osteocytes and chondrocytes.

Specifically, the present invention has a feature in embodying a single scaffold having a porous nanofibrous scaffold, which has a large surface area and is highly hydrophilic, thus enabling osteocytes to differentiate well, as a first layer, and a dense scaffold which is porous and has a dense structure, thus enabling chondrocytes to differentiate well, as a second layer.

In the present invention, the second layer scaffold, by having nanoscale matrices, has a large surface area and is significantly hydrophilic compared to the first layer scaffold. As a result, adhesion of cells, such as mesenchymal stem cells, which differentiate the osteocytes, to the nanofibrous matrices is enhanced, and at the same time, cell-to-cell contact is weakened, thereby accelerating osteogenesis of the mesenchymal stem cells. In contrast, the first layer scaffold is porous but has a dense surface, and thus has a smaller surface area and is less hydrophilic compared to the second layer scaffold. Consequently, osteocyte-to-scaffold adhesion is weakened, and cell-to-cell contact is improved, and is thereby advantageous in cell aggregation and proliferation. Therefore, the first layer scaffold is appropriate for maintaining a phenotype of cells such as osteocytes, and for differentiating stem cells into osteocytes.

The present invention provides a novel scaffold manufactured in an integrated form having a structure in which such first layer, the dense scaffold, and such second layer, the nano fibrous scaffold, are laminated.

The second layer nano fibrous scaffold can be made by mixing a certain amount of a biodegradable polymer and camphene, whereas the first layer dense scaffold includes no camphene.

The present description describes that the second layer nanofibrous scaffold includes camphene and the biodegradable polymer at a ratio of 1:1 to 12:1, specifically 4:1.

When camphene is included at a greater ratio, the pore structure may collapse, whereas when a lesser amount of camphene is included, it would be difficult to form the nano fibrous structure.

In the present invention, the first layer dense scaffold and the second layer nanofibrous scaffold may include the same biodegradable polymer. Rather than manufacturing the first and second layer scaffolds separately and binding them together, the present invention has a feature in that scaffolds having different pore structures are manufactured simultaneously in a series of steps, and that the same biodegradable polymer may be used. Additionally, due to the same biodegradable polymer, a double-layer scaffold can be easily manufactured.

Each of the first and second layer scaffolds may independently include one or more biodegradable polymers selected from the group consisting of polyhydroxybutyrate (PHB), polyhydroxyvalerate (PHV), polylactide, polyglycolide, polycaprolactone, polypropylene fumarate, polydioxanone, and a copolymer thereof.

Specifically, polylactide can be included.

The dense and nanofibrous scaffolds of the present invention have a pore size of 100 µm to 200 µm and porosity of 88% to 90%.

Compared to the dense scaffold, the nanofibrous scaffold, due to its nanoscale fibrous structure, can have a large amount of inter-fiber spaces, and thus may have a larger surface area and be highly hydrophilic.

Specifically, the surface area of the first layer dense scaffold is 2 m²/g to 4 m²/g. The surface area as such will facilitate cell-to-cell contact, e.g., between osteocytes, thereby leading to cell proliferation. In contrast, the surface area of the second layer nano fibrous scaffold is 12 m²/g to 14 m²/g.

The surface area as such will induce adhesion of the mesenchymal stem cells to the scaffold and promote differentiation and proliferation of the osteocytes. Additionally, with respect to hydrophilicity, the first layer dense scaffold has a 10° to 30° greater water contact angle than the second layer nanofibrous scaffold, and accordingly, the second layer nanofibrous scaffold may be more hydrophilic than the first layer dense scaffold.

Additionally, the present invention can provide a method for manufacturing the porous biphasic scaffold for osteochondral composite tissue regeneration.

The method for manufacturing the porous biphasic scaffold of the present invention comprises
(1) preparing a biodegradable polymer slurry to which camphene is added and a biodegradable polymer slurry to which camphene is not added; (2) preparing a mold in which metal salt particles of a uniform size are present; (3) injecting one of the slurries prepared in the first step into the mold prepared in the second step; and (4) injecting the other slurry into the mold.

Only one mold may be used in the above manufacturing method. In other words, the biodegradable polymer slurry can be injected into a mold partially, and then the biodegradable polymer slurry to which the camphene is not added can be filled in with the rest of the mold. In this process, some of the two polymer slurries may be mixed, forming a partially mixed biphasic scaffold.

The step (3) and/or (4) comprises lyophilizing each of the injected slurries and optionally dissolving the remained metal salt particles. The lyophilizing comprises partial or complete lyophilizing. Step (4) may be perfomed before completion of the lyophilizing of step (3).The dissolution of the metal salt particles may be by a solvent such as water.

A biphasic scaffold can be manufactured by separately manufacturing the first layer dense scaffold and the second layer nano fibrous scaffold and then combining the two; however, this requires further manufacturing process and may cause a problem such as involving a different external substance, *etc.* to completely combine the two solids. In contrast, the present invention can be manufactured via a series of continuous processes as above; and in this case, two layers completely combined with each other can be manufactured more simply and without involving an external substance.

A ratio of camphene to biodegradable polymer in the first is be 1:1 to 12:1 based on weight, specifically 4:1, but is not limited thereto.

In the manufacturing method, the metal salt particles is sodium chloride (NaCl) particles, but are not limited thereto. Additionally, a diameter of the metal salt particle is 100 µm to 150 µm.

In terms of the manufacturing method of the present invention, a solvent used for preparing the slurry is chloroform, 1,4-dioxane, dichloromethane, acetone, trifluoroethanol, hexafluoropropanol, or a combination thereof, specifically a combination of chloroform and 1,4. dioxane.

### [Advantageous Effects]

The scaffold of the present invention having the porous nanofibrous scaffold, which has a large surface area and is highly hydrophilic, thus enabling osteocytes to differentiate well, and the dense scaffold, which is porous and has a dense structure, thus enabling chondrocytes to differentiate well, embodied in an integrated form can be applied to an osteochondral composite tissue to simultaneously promote regeneration of the osteocytes and chondrocytes.

### [Description of the Drawings]

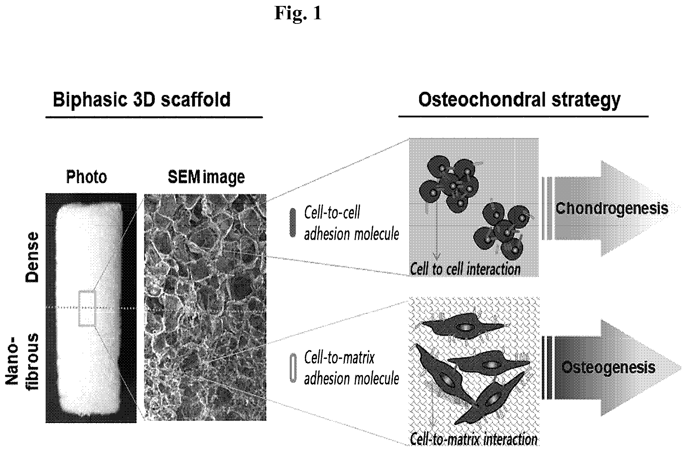

Fig. 1 is a schematic diagram showing the scaffold of the present invention for the osteochondral composite tissue engineering and a drawing showing the application strategy thereof.
Fig. 2 is a drawing and graphs showing the characteristics of the first layer dense scaffold and the second layer nanofibrous scaffold: (a) SEM images showing the morphology of the porous biphasic scaffold, (b) macro-porosity and pore size distribution, (c) specific surface area measured by a BET method, and (d) time-dependent changes in water contact angle.
Fig. 3 is drawings and graphs showing chondrocyte adhesion to the first layer dense scaffold or the second layer nanofibrous scaffold: (a) expression of integrin β1 by RT-PCR at 0 min, 30 min, 60 min, and 120 min, and the quantified intensity (b) cell adhesion number, and (c) CCK viability at 24 h.
Fig. 4 is a graph showing osteocyte proliferation measured by CCK assay up to 14 days.
Fig. 5 is drawings and graphs showing cell-to-cell interaction assay: (a) SEM morphological difference of the first layer dense-surfaced or the second layer nanofibrous-surfaced matrices, (b) representative CLSM images of immune-stained cells at day 7 (blue: nucleus, red: α-actin, and green: Col II), (c) DAPI-stained nuclei number, and (d) Col II expression intensity assayed from CLSM images.
Fig. 6 is graphs showing measured maintenance of chondrocyte phenotypes: (a) expression of chondrocyte-related genes, SOX-9, Col 11, and Aggrecan, by *q*RT-PCR at days 7 and 14, and (b) GAG production as quantified by the Alcian blue stain assay.
Fig. 7 is drawings and a graph showing MSC adhesion to the first layer dense scaffold or the second layer nanofibrous scaffold: (a) representative CLSM images of immune-stained cells (blue: nucleus, and red: α-actin), and (b) cell adhesion number measured at various initial time points.
Fig. 8 is drawings and a graph showing an analysis of the MSC proliferation: (a) representative CLSM images of cells at day 14, and (b) cell proliferation measured by CCK assay up to 14 days.

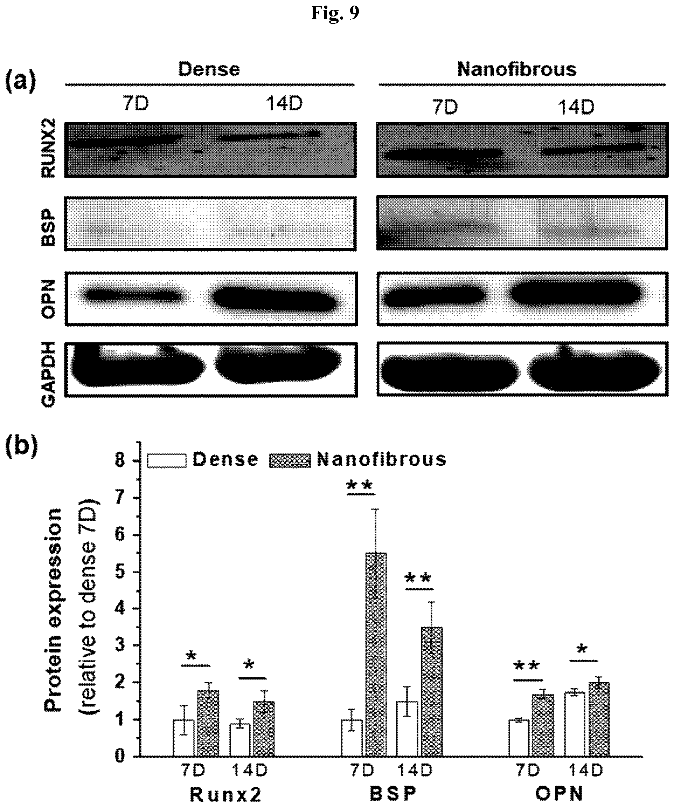

Fig. 9 is drawings and a graph showing osteogenesis of MSC: (a) osteogenic protein expressions (OPN, Runx2, and BSP) analyzed by Western blot at days 7 and 14, and (b) the intensities quantified by ImageJ (data normalized to a group Dense 7D).
Fig. 10 is a graph showing mineralization of MSCs.

### [Mode for Invention]

Hereinafter, the present invention will be described in more detail with reference to the following Examples. However, these Examples are for illustrative purposes only, and the invention is not intended to be limited by these Examples.

### Example 1: Preparation of dense-surfaced and nanofibrous-surfaced 3D scaffolds

Dense and nanofibrous 3D scaffolds were fabricated with poly-L/D-lactide (PLDLA; LESOMER@LR 708, Evonic).

While dissolving PLDLA at 3 wt% in a co-solvent of chloroform/1,4-dioxane (1:4), two types of the PLDLA slurries were prepared: one solution including 1.2 g of camphene and another solution without camphene added. NaCl particles (100 µm to 150 µm) were tamped to a plastic mold, and the PLDLA slurry to which camphene was not added was filled in the mold and was then frozen overnight at -20°C. The camphene-added PLDLA slurry was added to the mold having the solidified PLDLA and then was frozen overnight at -20°C.

Then, an additional freeze-drying was performed at -60°C, and the remaining NaCl particles were dissolved to obtain a gigantic porous biphasic scaffold structure.

### Experimental Example 1: Physicochemical properties assays

### 1-1. SEM image observation

The morphology of the scaffolds was observed by scanning electron microscopy (SEM, S-3000H, Hitachi, Japan). The porosity and pore size distribution of the scaffolds were analyzed using a mercury porosimeter (PM33, Quantachrome, USA). A specific surface area was measured by the N₂ adsorption/desorption (Quantachrome, USA) based on the Brunauer-Emmett-Teller (BET) method.

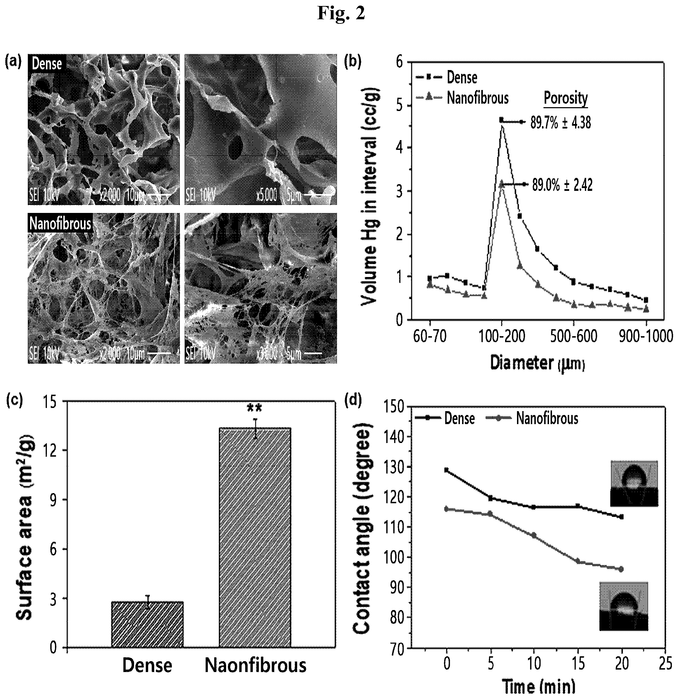

As a result of the observation of overall morphologies of the scaffolds using the SEM image, both scaffolds presented similar macroporous structures comprised of interconnected pores with sizes around 100 µm to 200 µm, as shown in Fig. 2a.

The internal morphologies of the two scaffolds were revealed to be significantly different. The phase separation process induced by the use of camphene allowed the creation of a highly nano fibrous morphology while maintaining the overall macropore configuration, whereas a smooth and dense pore morphology was produced in the absence of camphene. The total porosity of the artificial scaffolds was not much different based on the result of mercury intrusion porosimetry; and the total porosity of the dense and nanofibrous scaffolds was 89.7% and 89.0%, respectively (Fig. 2b).

In light of the SEM observation, the pore size distribution was similar in both scaffolds, with pores mainly peaking at 100 µm to 200 µm. However, the nanofibrous scaffolds also contained a huge amount of inter-fiber spaces (submicron to a few microns) due to the nanofibrous structure, which might not be detected by the mercury intrusion porosimetry.

### 1-2. Contact angle assays

The water affinity of the scaffolds was tested by measuring the water contact angle using a Phoenix300 analyzer. The water droplet images made onto the scaffold were observed using a viewing system until the equilibrium shape was achieved. Data was recorded for up to 20 min, and five samples were tested for each group. The specific surface area was measured by the N₂ adsorption/desorption (Quantachrome, USA) based on the Brunauer-Emmett-Teller (BET) method (Fig. 2c), and the hydrophilicity of the scaffolds was also determined by contact angle measurement (Fig. 2d).

The water contact angle decreased gradually with time for both scaffolds due to the time-sequenced water permeation behavior. The nano fibrous scaffolds showed much lower contact angles than the dense scaffolds, suggesting the water molecules should react with the nanofibrous surface of scaffolds more quickly than the dense surface. Due to the higher surface area, the nanofibrous surface could provide more reaction sites to water molecules, leading to an easier penetration of water into the pore channels.

Combining the results of Experimental Examples 1-1 and 1-2, the macroporous 3D scaffolds with a unique nanofibrous internal structure were produced to have similar porosity and pore configuration to the conventional dense-surfaced counterpart. Additionally, the nanofibrous structure significantly increased the surface area and water permeation of scaffolds. This behavior should play an important role in many biological interactions of scaffolds in body fluid or culture medium with protein and cells, and thus allowing control of cell and tissue behaviors.

### Experimental Example 2: Isolation of cells and culture

Primary chondrocytes (hereinafter, CCs) and mesenchymal stem cells (hereinafter, MSCs) were isolated from 5-week-old Sprague Dawley rats (180 g to 200 g) to test the chondrogenesis and osteogenesis ability on the scaffolds. The animal experiment followed the guidelines approved by the Animal Care and use Committee of Dankook University.

The CCs were isolated from the resting zone of the costochondral cartilage growth plate and rib region, as described elsewhere. Dissected rib cages were cut into small pieces and digested in 10 mL of DPBS containing 0.25% EDTA and incubated for 20 min at 37°C. The digested pieces were then incubated in 0.25% trypsin (GIBCO) for 1 h and in 0.2% collagenase type 2 for 3 h. The digested tissues were centrifuged at 2000 rpm followed by filtration with a sterile 45 µm sieve filter (BD Bioscience). The CCs were plated at a density of 2.5 × 10⁴ cells/cm² and cultured in Dulbecco's modified Eagle's medium (DMEM) with high glucose containing 10% fetal bovine serum (FBS), 1% penicillin/streptomycin, 50 µg/mL ascorbic acid, and 1% ITS premix (Sigma). The third passage of confluent cells was harvested for seeding onto scaffolds.

The MSCs were obtained from bone marrow of the tibia and femoral region. The harvested product was centrifuged, and the supernatant was collected and suspended within a culture flask containing a normal culture medium (minimal essential medium (α-MEM) supplemented with 10% FBS, and 100 U/mL penicillin and 100 mg/mL streptomycin). After incubation for 1 day, the medium was refreshed and cultured until the cells reached near confluence. The cells were subcultured with trypsinization and maintained in normal culture conditions. Second or third passage cells were harvested for seeding on scaffolds. The two types of primary cultured cells were incubated at 37°C in a humidified atmosphere of 5% CO₂ with media changes every 2 to 3 days.

### Experimental Example 3: Chondrocyte assays on scaffolds

### 3-1. Cell-to-scaffold adhesion and proliferation

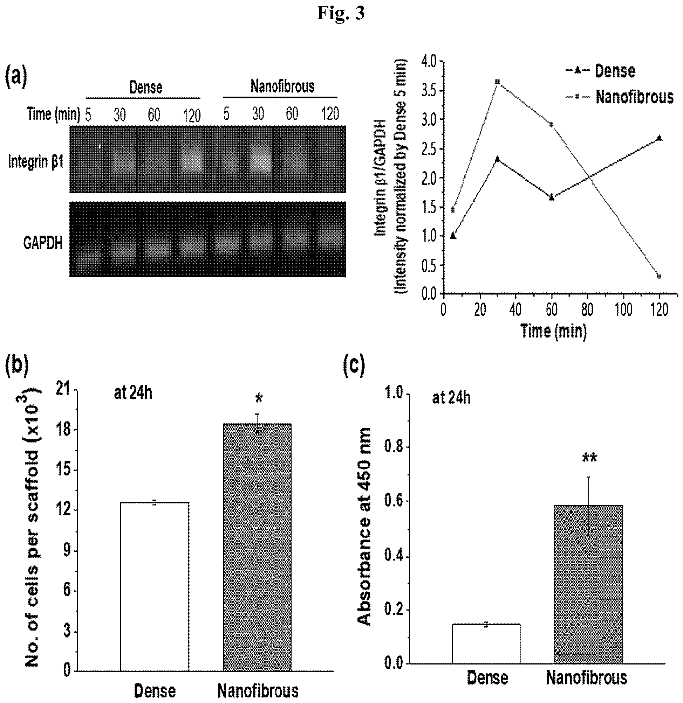

Rat primary CCs were used to examine the initial adhesion, proliferation, and maintenance of CC phenotypes onto dense and nanofibrous scaffolds. The initial cell-to-scaffold adhesion was observed by the gene expression of integrin β1, which transmits signals between the extracellular matrix and actin cytoskeleton (Fig. 3a). Additionally, the expression level of integrin β1 was analyzed at very early time points (5 min, 30 min, 60 min, and 120 min).

CCs were seeded on the scaffolds at a density of 5 × 10⁵ cells per 3D scaffold (1 mm height × 4 mm diameter). The gene expression of integrin β1 was confirmed by gel-based qPCR. Glyceraldehyde 3-phosphate dehydrogenase (GAPDH) was used as a control.

mRNA was extracted by directly dipping the scaffolds in lysis buffer (300 µL), and cDNA was synthesized from the total extracted RNA (1 µg) using an RT-Pre mix (Bioneer Ltd.). 2 µL cDNA was subjected to PCR amplification (Applied Biosystems Veriti Thermal Cycle) using specific designed primers (BLAST, NCBI) with a pre-mixed PCR kit (Bioneer, Daejeon, Korea). The PCR reactions were conducted using 40 cycles at 95°C for 30 sec, 58°C for 30 sec, and then 75°C for 60 sec. Experiments were performed in triplicate. Sample band intensity was quantified using Gene tools ver 4.01 software (Syngene UK), and normalized to that of GAPDH transcripts.

The adhesive cell number was indirectly calculated by counting the unattached cell number using a hemocytometer. For the cell proliferation test, the CCK-8 cell counting kit (Dojindo Molecular Technologies) was used. 2.5 × 10⁴ cells were seeded to a 3D scaffold and then cultured for 1, 3, 7, and 14 days. At each time, the samples were transferred to new 96-well plates and washed three times with PBS, replacing the culture media with a working CCK-8 solution. The reagent was left to react for 2 h at 37°C, and then an optical density was measured as absorbance at 450 nm (Biorad iMark reader).

In the nano fibrous scaffold, the gene expression peaked as early as 30 min with a slight decrease with time, whereas in the dense scaffold, the expression level appeared to increase more at a later time point (the highest at 120 min).

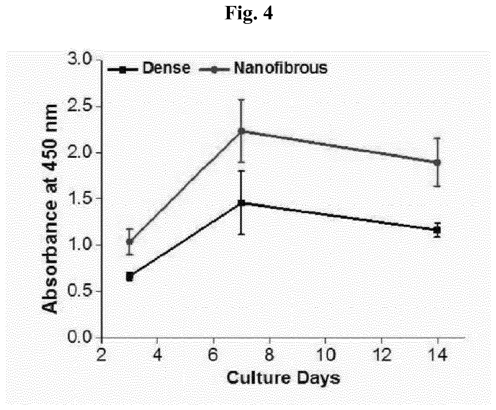

As shown in Fig. 3b, the cell adhesion number onto nanofibrous scaffolds showed a significantly higher (about 1.5 times) value than that of dense scaffolds. Additionally, as shown in Fig. 3c, the cell viability on nanofibrous scaffolds as measured by a CCK assay was significantly higher than that on dense scaffolds. The cells grow well on both scaffolds with comparable cell growth kinetics (Fig. 4).

It is considered that the higher surface area of nanofibrous scaffolds could provide increased cellular adhesion sites to cells. This cell adhesion event significantly enhanced in the nanofibrous scaffolds can alter the subsequent cellular behaviors such as increased cell spreading and early differentiation, which has been reported to be detrimental to the maintenance of the chondrogenic phenotypes.

### 3-2. Cell-to-cell interaction assays

To accurately determine the cell-to-cell contact, thin 2D films which have the same surface nanotopology as the 3D scaffolds were prepared. The PLDLA slurry was prepared as described in Example 1. In the case of the nano fibrous structured membrane, the camphene-added PLDLA slurry was used. The completely dissolved slurries were poured into a Teflon mold to freeze under liquid nitrogen and then freeze-dried for 3 days. The cell-to-cell interaction was examined by the aggregated colonies, and the number of cells at the aggregated colony region was counted.

Additionally, the expression of collagen type II (Col II) was examined as an indication of cell-to-cell contact of CCs. For this, 2 × 10⁴ cells were seeded onto each 2D membrane (16 mm diameter) for a culture period of 7 days. The samples were then fixed in 4% PFA and treated with 0.2% triton X-100. After washing with PBS, samples were treated with Alexa Fluor-546 phalloidin (Invitrogen) to stain α-actin and 4',6-diamidino-2-phenylindole (DAPI; Invitrogen) to stain nuclei. For immunofluorescence staining, primary antibody mouse monoclonal anti-collagen type II antibody (SC-52658, 1:500), and secondary antibody rabbit anti-mouse FITC were used. Images were visualized under a confocal laser scanning microscope (CLSM: Zeiss 700) and analyzed by Zen 2009 software.

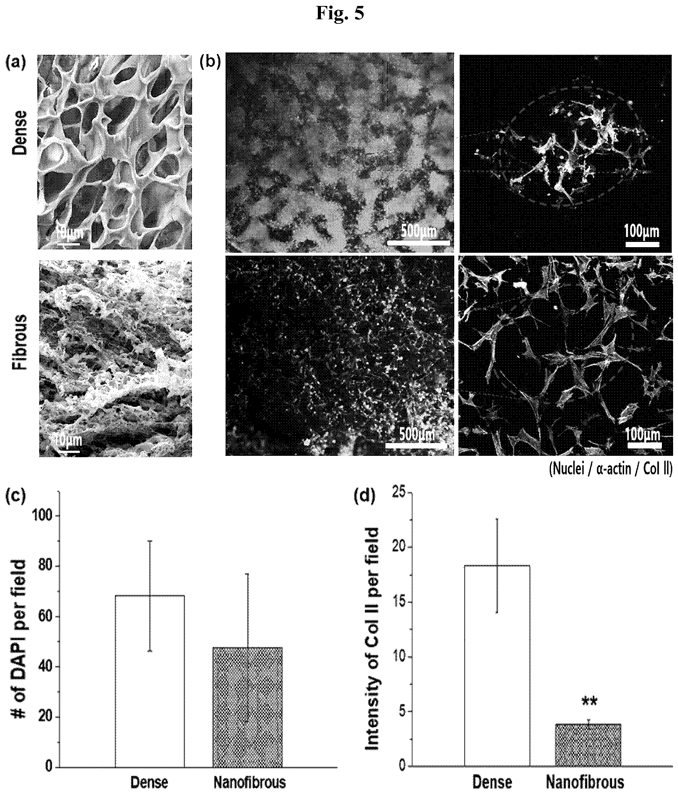

The cell-to-cell interactions of CCs were examined by the morphological change and the Col II expression. For more accurate observation of the surface-nanostructure-dependent cellular interactions, the scaffolds were prepared in a flat membrane type which led to a development of morphology with less pronounced macropore channels, but more contrasted dense or nanostructured surface topology (Fig. 5a).

Based on the cell-to-scaffold interaction results, the dense scaffolds are thought to offer more cell-to-cell interactions than the nanofibrous scaffolds. Specifically, the CCs cultured for 7 days showed clearly different behaviors. On the dense sample, the CCs were present unevenly, showing an irregular pattern-like circular arrangement of cell aggregates, whereas the cells on the nanofibrous sample were distributed more evenly without aggregation, based on optical microscopic images (Fig. 5b). Closer examination under a confocal microscope revealed that the cells on the dense sample were closely packed with limited cytoskeletal processes, whereas those on the nano fibrous sample were highly extensive and presented minimal aggregation. The micro-mass morphology of the aggregated CCs observed on the dense scaffolds was similar to that found in native cartilage tissues. This morphological development of CCs is closely related with the maintenance of the chondrocyte phenotype and the maturation into cartilage-like tissue. As such, while the cell numbers counted in the image for each field were comparable on both surfaces, the cells on a dense surface were less homogeneous in a more aggregated form (Fig. 5c). Of note, the intensity of Col II was significantly higher (about 7 times) on the dense surface than on the nanofibrous surface (Fig. 5d). Col II is considered as a representative marker of cell-to-cell contact of CCs. The collective results demonstrate that the dense scaffolds are able to enhance the cell-to-cell interactions at the cost of cell-matrix adhesion, which leads to formation of round-shaped cells with few actin filaments and ultimately the development of micro-mass cellular constructs.

### 3-3. Chondrogenesis of chondrocyte: gene expression

Chondrogenesis was determined by the expression of chondral-related genes (SOX-9, COL-11, and Aggrecan) after culturing the cells in chondrogenic media for 7 days and 14 days. The mRNA was isolated using RNeasy Minikit (Qiagen, 74106) according to the manufacturer's instructions. Reverse transcription was performed using a thermal cycler (HID Veriti^{®}96-Well Thermal Cycler, Applied Biosystems, 4479071), and the obtained cDNA was quantified with a UV-Vis Spectrophotometer (Nanodrop 2000, Thermo Scientific). Gene expression levels were evaluated by a real-time PCR equipment (StepOneTMPlus, Applied Biosystems) using SensiMix^{™} SYBR Kit (Bioline, QT605). Primers for the gene amplification are summarized in Table 2.

**[Table 2]**

| Gene | | Sequence |
|---|---|---|
| GAPDH | Forward primer | 5'CTGGAAGATGGTGATGG3' |
| | Reverse primer | 5'GATTTGGTCGTATTGGGCG3' |
| Aggrecan | Forward primer | 5'TCGCAAGTCCCTTCCACATC3' |
| | Reverse primer | 5'TCAAGGCGTCCTGAAGTGTC3' |
| Collagen type II | Forward primer | 5'GCTGGTGCACAAGGTCCTAT3' |
| | Reverse primer | 5'AGGGCCAGAAGTACCCTGAT3' |
| Sox9 | Forward primer | 5'CCAGCAAGAACAAGCCACAC3' |
| | Reverse primer | 5'CTTGCCCAGAGTCTTGCTGA3' |

The PCR was initiated with an activation step of 20 sec at 95°C, followed by 40 cycles of denaturation (15 sec, 95°C), annealing (30 sec, 60°C), and extension (30 sec, 72°C). Relative gene expression levels for target genes were normalized to the endogenous GAPDH gene using the comparative Ct method (2^{-ΔΔCt}) as previously mentioned.

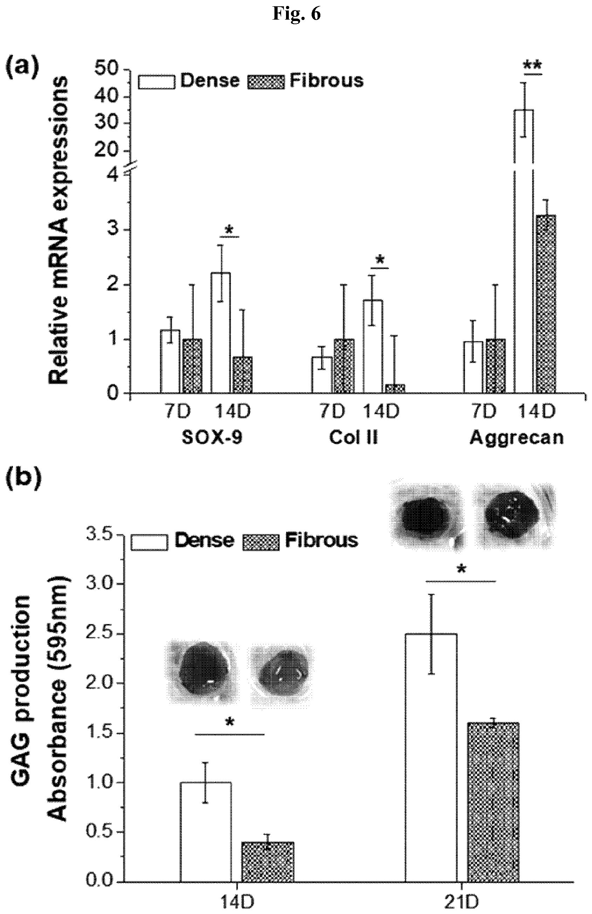

The expression of genes related with the CC phenotype, such as SOX-9, Col 11, and Aggrecan, were examined at days 7 and 14 by RT-PCR (Fig. 6a). All of the genes were expressed more on the dense scaffolds than on the nanofibrous scaffolds, particularly at day 14. The most notable expression was found in Aggrecan, in which the dense scaffolds showed up to 35-fold up-regulation of the gene at day 14 compared to the nanofibrous scaffolds. While the SOX-9 and Col II are known as transcription factors expressed at relatively early stages mainly associated with the maintenance of the CCs, the Aggrecan is produced at relatively later stages and is considered as a major proteoglycan in cartilage ECM. While being indicative of increased cell-to-cell interactions, the Aggrecan expression is also involved in the cell-to-ECM molecular interactions in cartilage due to its ability to bind hyaluronan. Therefore, the expression of a substantial level of the Aggrecan by the cells during the culture of 14 days on the dense-surfaced scaffolds suggested the possible functional development (*i*.*e*., cartilage-like ECM synthesis) of the cellular constructs.

### 3-4. Maturation of chondrocytes

Glycosaminoglycan (GAG) production was observed by Alcian blue staining and quantitatively analyzed at 14 days and 21 days. The scaffolds were rinsed with PBS followed by fixation in 4% PFA at 4°C for 10 min. The scaffolds were transferred to a 1% Alcian blue (Sigma) solution, diluted in 3% acetic acid, and incubated at room temperature for 30 min. The scaffolds were rinsed in D.W. three times followed by incubation in 1% SDS for 30 min to solubilize the Alcian blue stain. The absorbance of the eluting solution was measured at 595 nm using an iMark reader.

As a result of further analyzing the production of key cartilage matrix molecule glycosaminoglycan (GAG) by means of staining the cells with Alcian blue after 14 and 21 days' culturing (Fig. 6b), the cellular constructs cultured on dense-surfaced scaffolds revealed much darker blue color stains than those on the nanofibrous scaffolds at both culture periods, thereby presenting significantly higher GAG production. The quantification result of the Alcian blue dye amount showed higher levels on the dense scaffolds than on the nanofibrous scaffolds. This is a clear indication that the CCs cultured on the dense-surfaced scaffolds become more functional to synthesize abundant GAG molecules, thus implying more potential for the cartilage regeneration when compared to the cells cultured on the nanofibrous scaffolds.

### Experimental Example 4: Mesenchymal stem cell (MSC) assays on scaffolds

### 4-1. Cell-to-scaffold adhesion and proliferation

The MSCs were used to examine their interactions with two different types of scaffolds, including cell adhesion, growth, and osteogenic differentiation. The initial adhesion behavior of MSCs onto the scaffolds was investigated by counting the attached cell numbers. In brief, 5 × 10⁵ cells were seeded onto each 3D scaffold and cultured for 2 h, 4 h, 8 h, and 16 h, and the number of attached cells was determined by measuring the remaining cells in the media using a hemocytometer.

To observe the morphology, the cells were fixed in 4% PFA for 10 min at room temperature, and then permeabilized with 0.1% triton × 100 for 3 min, followed by PBS washing, and then blocking with 1% BSA for 30 min. The cells were stained with Alexa fluor-555 phalloidin for 45 min at room temperature followed by PBS washing, and the nuclei were stained with DAPI for 5 min. The cell images were then visualized with CLSM.

For the proliferation test, the CCK-8 cell counting kit was used. 2.5 × 10⁴ cells were seeded onto each 3D scaffold and cultured for 1, 3, 7, and 14 days. As the protocol of the cell adhesion assay, the cells were fixed in 4% PFA for 10 min at room temperature, and then permeabilized with 0.1% triton X 100 for 3 min, followed by PBS washing, and then blocking with 1% BSA for 30 min. The cells were stained with Alexa fluor-555 phalloidin for 45 min at room temperature followed by PBS washing, and the nuclei were stained with DAPI for 5 min. The cell images were visualized with CLSM.

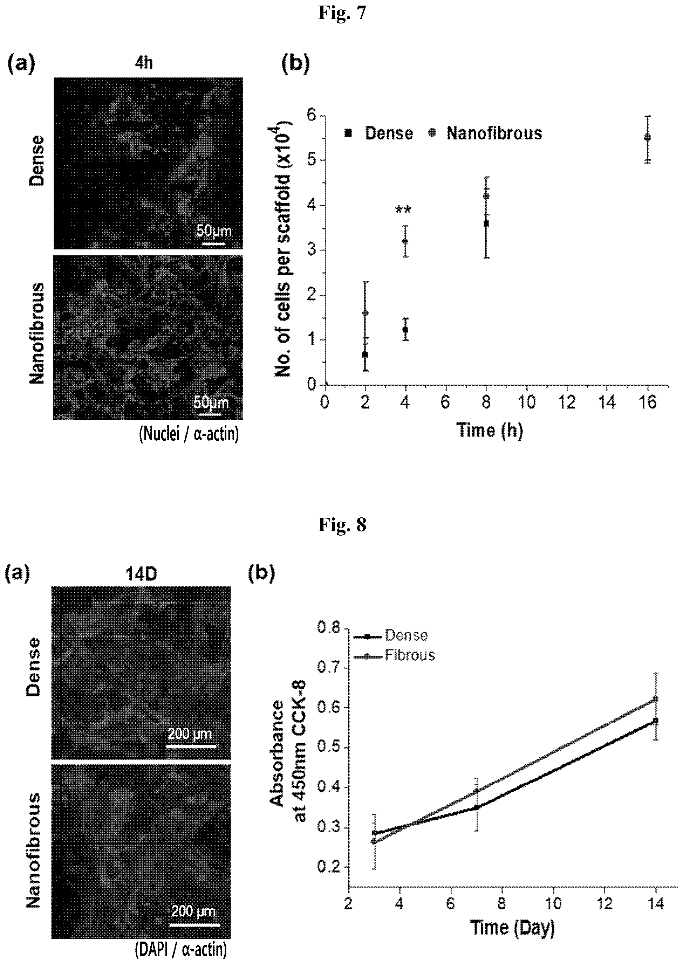

Compared to dense scaffolds, the nanofibrous scaffolds showed more cells and better spreading (Fig. 7a). Additionally, the cell adhesion recorded up to 16 h showed significantly higher levels on nanofibrous scaffolds than on dense scaffolds, particularly until 4 h, by an about 3-fold difference, suggesting that the nanofibrous scaffolds accelerated the adhesion events of MSCs (Fig. 7b). The cell proliferation rate measured up to 14 days was shown to be similar between the two scaffolds (Fig. 8).

### 4-2. Osteogenesis of MSCs: protein expression

The osteogenesis of the MSCs was identified by the expression of some key osteogenic proteins, (e.g., Runx-2, BSP, and OPN) by Western blot. GAPDH was used as a control. Protein extracts from the MSCs cultured in an osteogenic medium were prepared by a RIPA buffer (PRO-PREP^{™} Protein Extraction Solution, iNtRON, South Korea). Protein samples were resolved on 10% SDS (sodium dodecyl sulfate)-polyacrylamide gel by boiling at 100°C for 4 min in a 2x sample buffer followed by transblotting to nitrocellulose membranes (Millipore).

The membranes were blocked with 5% BSA in Tris-buffered saline with 0.1% Tween-20 and then probed with proper primary antibodies (rabbit anti-RUNX-2 antibody SC-10758 at 1:500, mouse anti-OPN antibody SC-20788 at 1:500, rabbit anti-BSP antibody SC-292394 at 1:1000, and mouse anti-GAPDH antibody SC-25778 at 1:500).

Secondary antibodies were goat anti-mouse and goat anti-rabbit FITC antibodies. All antibodies were purchased from Santa Cruz Inc. The blotted membranes were then incubated with Horseradish peroxidase (HRP)-conjugated secondary immunoglobulin G (Invitrogen), and band signals were detected using an enhanced chemiluminescent (ECL) detection reagent (Pierce, Rockford, USA). ECL treatment membranes were visualized using an LAS-1000 mini image analyzer (GE, USA), and the band intensities were quantitatively analyzed by ImageJ software.

As a result of observing the key osteogenic proteins such as Runx-2, BSP, and OPN, the relatively early osteogenic marker, Runx-2, was expressed at a significantly higher level on the fibrous scaffolds compared to the dense scaffolds at both 7 days and 14 days. This trend was also observed for the relatively late osteogenic markers, BSP and OPN.

### 4-3. Maturation of MSCs

Cellular mineralization was assessed by means of an Alizarin red S (ARS) staining at 28 days' culture in an osteogenic induction medium. Cells were washed with PBS and fixed with 10% PFA for 15 min at room temperature. Cells were stained with a 40 mM ARS solution (pH 4.2) and incubated for 10 min at room temperature with gentle shaking. The media were aspirated, and the scaffolds were washed three times. The stained samples were captured using a digital camera. For quantification, the ARS stain was de-stained using 10% cetylpyridinium chloride (CPC) in 10 mM sodium phosphate buffer (pH 7.0) for 15 min at room temperature. Aliquots of extracts were diluted 10-fold in 10% CPC solution, and absorbance of the elucidated solution was detected at 562 nm using a microplate reader.

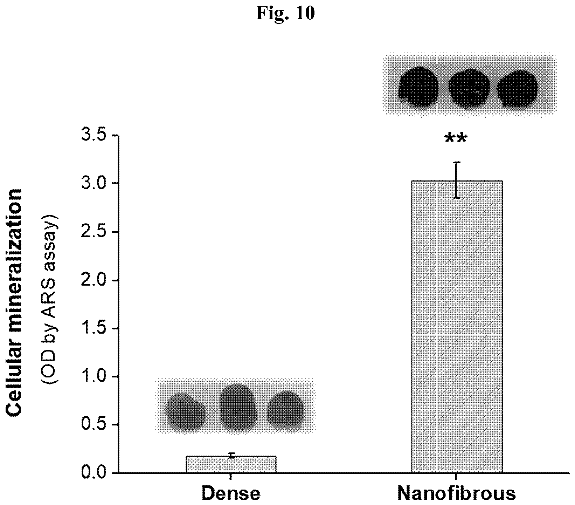

As a result of investigating mineralization of the osteogenic cells through the calcium deposition (Fig. 10), the ARS staining showed a much darker red color on the nano fibrous scaffolds than on the dense scaffolds. When quantified, the difference was as high as 6-fold.

Consequently, it was revealed that the osteocytes showed better adhesion and proliferation on the nanofibrous scaffolds than on the dense scaffolds.

## Claims

1. A porous biphasic scaffold for osteochondral composite tissue regeneration, comprising:
a first layer scaffold having a dense structure for cartilage regeneration comprising a biodegradable polymer; and
a second layer scaffold having a nanofibrous structure for bone regeneration comprising a biodegradable polymer and camphene;
wherein the surface area of the first layer scaffold is 2 m²/g to 4 m²/g and that of the second layer scaffold is 12 m²/g to 14 m²/g.

2. The scaffold of claim 1, wherein the first and second layer scaffolds comprise the same biodegradable polymer.

3. The scaffold of claim 1 or 2, wherein each of the first and second layer scaffolds independently comprise one or more biodegradable polymers selected from the group consisting of polyhydroxybutyrate (PHB), polyhydroxyvalerate (PHV), polylactide, polyglycolide, polycaprolactone, polypropylene fumarate, polydioxanone, and a copolymer thereof.

4. The scaffold of any one of claims 1 to 3, wherein the second layer scaffold is more hydrophilic than the first layer scaffold.

5. The scaffold of any one of claims 1 to 4, wherein the first layer scaffold has a 10° to 30° greater water contact angle than the second layer scaffold.

6. The scaffold of any one of claims 1 to 5, wherein the first layer and second layer scaffolds have a pore size of 100 µm to 200 µm.

7. A method for preparing a porous biphasic scaffold for osteochondral composite tissue regeneration, comprising:
(1) preparing a biodegradable polymer slurry to which camphene is added and a biodegradable polymer slurry to which camphene is not added;
(2) preparing a mold in which metal salt particles of a uniform size are present;
(3) injecting one of the slurries prepared in the first step into the mold prepared in the second step; and
(4) injecting the other slurry into the mold.

8. The method of claim 7, step (3) and/or (4) comprising lyophilizing each of the injected slurries and optionally dissolving the remained metal salt particles.

9. The method of claim 7 or 8, wherein a ratio of camphene to biodegradable polymer in the first step is 1:1 to 12:1 based on weight.

10. The method of any one of claims 7 to 9, wherein the metal salt particle is a sodium chloride (NaCl) particle.

11. The method of any one of claims 7 to 10, wherein the metal salt particle has a diameter of 100 µm to 150 µm.

12. The method of any one of claims 7 to 11, wherein a solvent used for preparing the slurry is chloroform, 1,4-dioxane, dichloromethane, acetone, trifluoroethanol, hexafluoropropanol, or a combination thereof.

## Patentansprüche

1. Poröses biphasisches Gerüst zur osteochondralen Verbundgeweberegeneration, umfassend:
ein erstes Schichtgerüst mit einer dichten Struktur zur Knorpelregeneration, umfassend ein biologisch abbaubares Polymer; und
ein zweites Schichtgerüst mit einer Nanofaserstruktur zur Knochenregeneration, umfassend ein biologisch abbaubares Polymere und Camphen;
wobei die Oberfläche des ersten Schichtgerüsts 2 m²/g bis 4 m²/g beträgt und die des zweiten Schichtgerüsts 12 m²/g bis 14 m²/g beträgt.

2. Gerüst nach Anspruch 1, wobei das erste und das zweite Schichtgerüst dasselbe biologisch abbaubare Polymer umfassen.

3. Gerüst nach Anspruch 1 oder 2, wobei jedes von dem ersten und dem zweiten Schichtgerüst unabhängig ein oder mehrere biologisch abbaubare Polymere umfasst, die aus der Gruppe bestehend aus Polyhydroxybutyrat (PHB), Polyhydroxyvalerat (PHV), Polylactid, Polyglycolid, Polycaprolacton, Polypropylenfumarat, Polydioxanon und einem Copolymer davon ausgewählt sind.

4. Gerüst nach einem der Ansprüche 1 bis 3, wobei das zweite Schichtgerüst hydrophiler als das erste Schichtgerüst ist.

5. Gerüst nach einem der Ansprüche 1 bis 4, wobei das erste Schichtgerüst einen um 10° bis 30° größeren Wasserkontaktwinkel als das zweite Schichtgerüst aufweist.

6. Gerüst nach einem der Ansprüche 1 bis 5, wobei das erste Schicht- und das zweite Schichtgerüst eine Porengröße von 100 µm bis 200 µm aufweisen.

7. Verfahren zur Herstellung eines porösen biphasischen Gerüsts zur osteochondralen Verbundgeweberegeneration, umfassend:
(1) Herstellen einer biologisch abbaubaren Polymersuspension, der Camphen zugegeben wird, und einer biologisch abbaubaren Polymersuspension, der kein Camphen zugegeben wird;
(2) Herstellen einer Form, in der Metallsalzteilchen einer einheitlichen Größe vorliegen;
(3) Einspritzen einer der in dem ersten Schritt hergestellten Suspensionen in die in dem zweiten Schritt hergestellte Form; und
(4) Einspritzen der anderen Suspension in die Form.

8. Verfahren nach Anspruch 7, wobei Schritt (3) und/oder (4) ein Lyophilisieren von jeder der eingespritzten Suspensionen und optional ein Lösen der verbliebenen Metallsalzteilchen umfassen.

9. Verfahren nach Anspruch 7 oder 8, wobei ein Verhältnis von Camphen zu biologisch abbaubarem Polymer im ersten Schritt 1:1 bis 12:1 beträgt, bezogen auf das Gewicht.

10. Verfahren nach einem der Ansprüche 7 bis 9, wobei das Metallsalzteilchen ein Natriumchloridteilchen (NaCl-Teilchen) ist.

11. Verfahren nach einem der Ansprüche 7 bis 10, wobei das Metallsalzteilchen einen Durchmesser von 100 µm bis 150 µm aufweist.

12. Verfahren nach einem der Ansprüche 7 bis 11, wobei ein Lösungsmittel, das zum Herstellen der Suspension verwendet wird, Chloroform, 1,4-Dioxan, Dichlormethan, Aceton, Trifluorethanol, Hexafluorpropanol oder eine Kombination davon ist.

## Revendications

1. Echafaudage biphasique poreux pour la régénération de tissu composite ostéochondral, comprenant :
un échafaudage de première couche ayant une structure dense pour la régénération de cartilage, comprenant un polymère biodégradable ; et
un échafaudage de deuxième couche ayant une structure nanofibreuse pour la régénération osseuse, comprenant un polymère biodégradable et du camphène ;
dans lequel la surface spécifique de l'échafaudage de première couche est de 2 m²/g à 4 m²/g et celle de l'échafaudage de deuxième couche est de 12 m²/g à 14 m²/g.

2. Echafaudage selon la revendication 1, dans lequel les échafaudages de première couche et de deuxième couche comprennent le même polymère biodégradable.

3. Echafaudage selon la revendication 1 ou 2, dans lequel chacun des échafaudages de première couche et de deuxième couche comprennent indépendamment un ou plusieurs polymères biodégradables choisis dans le groupe constitué par un polyhydroxybutyrate (PHB), un polyhydroxyvalérate (PHV), un polylactide, un polyglycolide, une polycaprolactone, un poly(fumarate de propylène), une polydioxanone, et leurs copolymères.

4. Echafaudage selon l'une quelconque des revendications 1 à 3, dans lequel l'échafaudage de deuxième couche est davantage hydrophile que l'échafaudage de première couche.

5. Echafaudage selon l'une quelconque des revendications 1 à 4, dans lequel l'échafaudage de première couche a un angle de contact avec l'eau supérieur de 10° à 30° à celui de l'échafaudage de deuxième couche.

6. Echafaudage selon l'une quelconque des revendications 1 à 5, dans lequel les échafaudages de première couche et de deuxième couche ont une taille de pores de 100 µm à 200 µm.

7. Procédé pour préparer un échafaudage biphasique poreux pour la régénération de tissu composite ostéochondral, comprenant :
(1) la préparation d'une bouillie de polymères biodégradables à laquelle du camphène est ajouté et d'une bouillie de polymère biodégradable à laquelle du camphène n'est pas ajouté ;
(2) la préparation d'un moule dans lequel des particules de sel métallique de taille uniforme sont présentes ;
(3) l'injection de l'une des bouillies préparées dans la première étape dans le moule préparé dans la deuxième étape ; et
(4) l'injection de l'autre bouillie dans le moule.

8. Procédé selon la revendication 7, dans lequel les étapes (3) et/ou (4) comprennent la lyophilisation de chacune des bouillies injectées et éventuellement la dissolution des particules de sel métallique restantes.

9. Procédé selon la revendication 7 ou 8, dans lequel le rapport en poids du camphène au polymère biodégradable dans la première étape est de 1/1 à 12/1.

10. Procédé selon l'une quelconque des revendications 7 à 9, dans lequel la particule de sel métallique est une particule de chlorure de sodium (NaCl).

11. Procédé selon l'une quelconque des revendications 7 à 10, dans lequel la particule de sel métallique a un diamètre de 100 µm à 150 µm.

12. Procédé selon l'une quelconque des revendications 7 à 11, dans lequel un solvant utilisé pour la préparation de la bouillie est le chloroforme, le 1,4-dioxane, le dichlorométhane, l'acétone, le trifluoroéthanol, l'hexafluoropropanol, ou une de leurs combinaisons.
